# EUROPÄISCHE PATENTSCHRIFT

(11) **EP 0 656 844 B1**
(45) Veröffentlichungstag und Bekanntmachung des Hinweises auf die Patenterteilung: **23.04.1997**
(21) Anmeldenummer: 93917629.3
(22) Anmeldetag: 23.07.1993
(51) Int. Cl.: B60S 1/16, F16C 11/04

(54) **KURBELTRIEB FÜR EINEN SCHEIBENWISCHERANTRIEB EINES KRAFTFAHRZEUGS**
CRANK MECHANISM FOR A WINDSCREEN WIPER DRIVE OF A MOTOR VEHICLE
COMMANDE A MANIVELLE POUR ENTRAINEMENT D'ESSUIE-GLACE SUR UN VEHICULE AUTOMOBILE

(30) Priorität: 27.08.1992 DE 4228493
(43) Veröffentlichungstag der Anmeldung: 14.06.1995
(73) Patentinhaber: ITT Automotive Europe GmbH, 60488 Frankfurt am Main (DE)
(72) Erfinder: RIENHARDT, Hans, Peter, D-74172 Neckarsulm (DE)
(86) Internationale Anmeldenummer: EP9301960
(87) Internationale Veröffentlichungsnummer: WO9405531

(56) Entgegenhaltungen:
- US-A- 3 262 038
- US-A- 3 462 179

## Beschreibung

Die Erfindung bezieht sich auf einen Kurbeltrieb einer Antriebsvorrichtung eines Scheibenwischers für ein Kraftfahrzeug, bestehend aus einem Hebel und einer über ein Drehgelenk damit verbundenen Lasche, wobei die Lasche exzentrisch an einem angetriebenen Getrieberad, insbesondere Schneckenrad des Getriebes, angelenkt ist und, der Hebel drehfest mit einer Wischerwelle verbunden oder verbindbar ist. Die Lasche und der Hebel bilden dabei einen Kniehebel, mit dessen Hilfe die Drehbewegung des Zahnrads bzw. Schneckenrads in eine Pendelbewegung der Wischerwelle umgesetzt wird. Dabei kann die drehfeste Verbindung zwischen dem Hebel und der Wischerwelle unmittelbar oder indirekt erfolgen.

Eine Kurbeltrieb dieser Art gemäß dem Oberbegriff des Anspruchs 1 ist aus US-A-3 262 038 bereits bekannt. Der Hebel und die Lasche werden dabei mittels eines separat hergestellten und zu montierenden Gelenkbolzen miteinander verbunden. Dies ist ein verhältnismäßig teuerer und aufwendiger Arbeitsgang.

Es liegt infolgedessen die Aufgabe vor, den Kurbeltrieb der eingangs genannten Art so weiterzubilden, daß sich die Herstellungskosten, insbesondere die Montagekosten verringern lassen.

Zur Lösung dieser Aufgabe wird erfindungsgemäß vorgeschlagen, daß der Kurbeltrieb gemäß dem Oberbegriff des Anspruchs 1 entsprechend dem kennzeichnenden Teil dieses Anspruchs ausgebildet ist. Die beiden Hälften der bajonettartigen Verbindung können an die Lasche und den Hebel unmittelbar angeformt bzw. daran ausgebildet werden. Man verbindet sie in der hierfür vorgesehenen Relativlage und bringt sie dann in eine ihre Montage an der Antriebsvorrichtung ermöglichende, einen anderen Winkel miteinander einschließende Lage. Dies reicht aus, um ein unerwünschtes Lösen der bajonettartigen Verbindung zu verhindern. Weil jede mögliche Relativlage der Lasche und des Hebels während des Betriebs der Scheibenwischvorrichtung von der Relativlage dieser Teile beim Herstellen der bajonettartigen Verbindung verschieden ist, kann sich letztere während des Betriebs der Scheibenwischvorrichtung nicht lösen. Aus dem Vorstehenden folgt aber, daß man diese Verbindung ohne Werkzeug herstellen kann und auch separate Teile entbehrlich sind. Dies reduziert nicht nur die Teilezahl, sondern auch die Herstellungskosten und die Montagezeit. Gegenüber einer bekannten Ausführung kann die Teilezahl im extremen Falle von 9 auf 2 gesenkt werden.

Bei einer bevorzugten Ausführungsform der Erfindung stehen die Lasche und der Hebel in der angekuppelten Montagestellung etwa in Verlängerung voneinander, d.h. das Kniegelenk ist beim Zusammenfügen von Lasche und Hebel zumindest im wesentlichen gestreckt.

Eine Weiterbildung der Erfindung ergibt sich aus Anspruch 3. Dort ist die Ausbildung der Elemente für die bajonettartige Verbindung im einzelnen ausgeführt. Weil auf diese Weise lediglich die gelenkige Verbindung zwischen Hebel und Lasche hergestellt wird, muß entsprechend Sorge dafür getragen werden, daß ein axiales Lösen der gelenkigen Verbindung nicht möglicht ist. Dies ist aber ohne besonderen Aufwand dadurch möglich, daß sich dieser Kniehebel in einem Gehäuseraum bewegt, der eine axiale Verschiebung des ganzen Kniegelenks oder auch nur eines Teils davon quer zur Bewegungsebene gar nicht zuläßt.

Eine weitere Variante der Erfindung ist dadurch gekennzeichnet, daß der Hebel an seinem anderen Ende eine insbesondere mit einer Sackbohrung versehene Hülse zur Aufnahme des inneren Endes der Welle aufweist. Die Wischerwelle muß aber in die Bohrung bzw. Sackbohrung nicht notwendigerweise eingesteckt oder eingepreßt werden, vielmehr kann man sich auch entsprechend umspritzen.

Am Hebel, der Hülse etwa gegenüberliegend, befindet sich in sehr vorteilhafter Weise ein balliges, innen an einem Gehäusedeckel der Antriebsvorrichtung abstützbares Stützglied. Es verhindert die Verschiebebewegung des Hebels quer zu seiner Bewegungsebene. Aufgrund der balligen Ausbildung ist die Reibung am Deckel auf ein Minimum reduziert. Dies gilt insbesondere dann, wenn man eine geeignete Werkstoffpaarung vorsieht.

Eine weitere Ausgestaltung der Erfindung ist Anspruch 6 zu entnehmen. Sie beinhaltet quasi indirekt, daß die Dicke der Lasche größer ist als diejenige des Hebels zumindest im Verbindungsbereich. Im übrigen sind die dort erwähnten randoffenen Einführungsschlitze selbstverständlich kongruent angeordnet. Der Vorteil dieser Laschenausbildung besteht darin, daß in jeder Arbeitsstellung dieses Kniegelenks ein Abheben der Lasche vom Hebel oder umgekehrt nicht möglich ist.

Diese Lasche kann in sehr vorteilhafter Weise gemäß Anspruch 7 weitergebildet werden. Das dort erwähnte ballige Stützglied entspricht demjenigen des Hebels und es hat auch die gleiche Aufgabe wie jenes.

Eine weitere Vereinfachung und Verbilligung sowie die Reduzierung auf die erwähnten zwei Teile erreicht man in weiterer Ausbildung der Erfindung dadurch, daß sich am hebelfernen Ende der Lasche ein quer zu ihrer Bewegungsebene vorstehender Lagerzapfen zum Eingriff in eine Lagerbohrung am Zahnrad, insbesondere Schneckenrad, befindet. In diesem Falle reichen also für diesen Kurbeltrieb in der Tat zwei Teile aus. Der Lagerzapfen greift hierbei in eine Lagerbohrung des Zahnrads mit entsprechendem Lagerspiel ein. Sie ist in bekannter Weise exzentrisch zur mittigen Lagerachse des Zahnrads angeordnet.

Der Hebel und die Lasche sind in besonders bevorzugter Weise aus Kunststoff gefertigt, insbesondere gespritzt.

Die Erfindung wird nachstehend anhand der Zeichnung näher erläutert. Die Zeichnung zeigt ein Ausführungsbeispiel der Erfindung. Hierbei stellen dar:
- Figur 1: eine Draufsicht auf den in eine Antriebsvorrichtung eines Scheibenwischers für einen in ein Kraftfahrzeug eingebauten Kurbeltrieb bei geöffnetem Kurbelgehäuse;
- Figur 2: in vergrößertem Maßstab eine Seitenansicht des Hebels dieses Kurbeltriebs;
- Figur 3: eine Draufsicht, und
- Figur 4: einen Schnitt durch diesen Hebel gemäß der Linie IV-IV der Figur 2;
- Figuren 5 und 6: Darstellungen der Lasche entsprechend den Figuren 3 und 2;
- Figuren 7 und 8: Schnitte gemäß Linien VII-VII bzw. VIII-VIII der Figur 6.

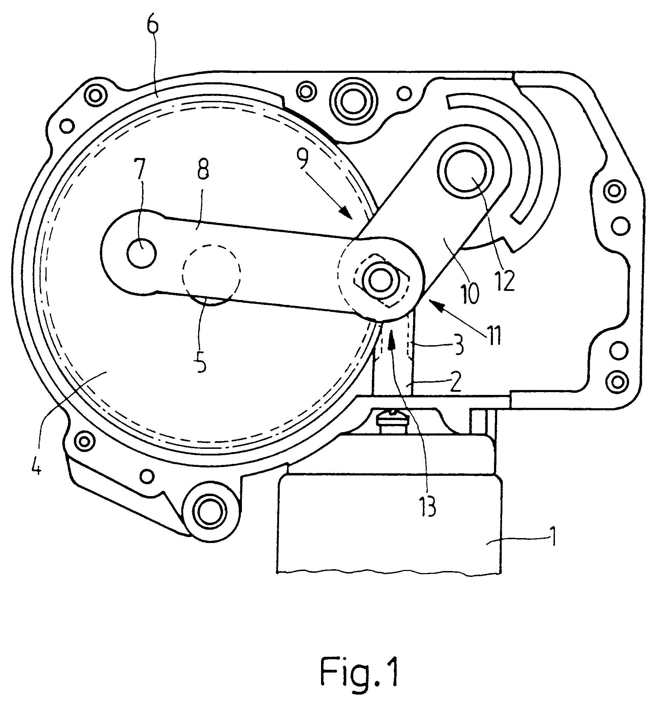

Die Antriebswelle 2 eines Elektromotors 1 trägt eine Schnecke 3, welche ein Schneckenrad 4 antreibt. Dieses ist mittels einer Welle 5 im Gehäuse drehbar gelagert. Ein zur Welle 5 exzentrischer Lagerzapfen 7 verbindet das als Schneckenrad 4 ausgebildete Getrieberad mit einer Lasche 8 eines Kurbeltriebs 9. Zu letzterem gehört noch ein Hebel 10 und beide zusammen bilden gemäß Figur 1 einen Kniehebel. Sie sind über ein Drehgelenk 11 miteinander verbunden, das nachstehend näher erläutert wird.

Am drehgelenkfernen Ende des Hebels 10 befindet sich die Wischerwelle 12. Sie durchsetzt das Gehäuse 6 nach außen hin und trägt an ihrem freien Ende in bekannter Weise den Wischarm. Ein die vorstehend erwähnten Elemente dieses Scheibenwischerantriebs abdeckender und teilweise die Elemente gegen eine Bewegung quer zur Bildebene sichernder Deckel ist in Figur 1 der Übersichtlichkeit wegen weggelassen.

Erfindungsgemäß sind die Lasche 8 und der Hebel 10 über eine bajonettartige Verbindung 13 gelenkig miteinander verbunden. Diese gestattet eine Verbindung ohne Zuhilfenahme irgendwelcher Werkzeuge, wobei sich diese Verbindung während des Betriebs nicht von selbst lösen kann. Dies hängt damit zusammen, daß sich die Lasche 8 und der Hebel 10 nur in einer genau vorgegebenen Montagestellung miteinander verbinden lassen, die von jeder Drehlage, welche dieser Kurbeltrieb 9 während des Betriebs einnimmt, verschieden ist.

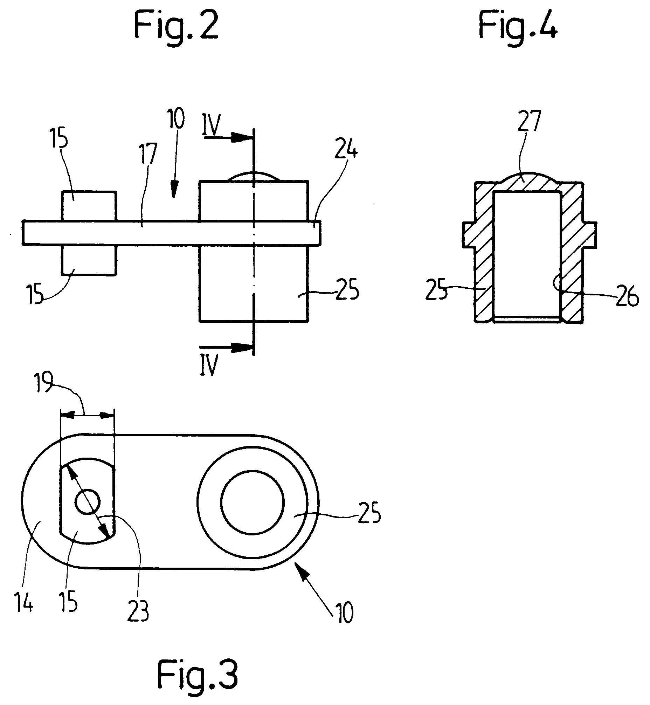

In dieser Montagestellung stehen beim Ausführungsbeispiel und in besonderer Ausgestaltung der Erfindung die Lasche 8 und der Hebel 10 in angekuppelter Stellung etwa in Verlängerung voneinander. Das heißt mit anderen Worten, daß die Lasche und der Hebel zunächst parallel zueinander ausgerichtet sind und dann eines der beiden Teile parallel zu sich gegen das andere hin verschoben wird, bis ein sich am einen Verbindungsende 14 des Hebels 10 quer zu dessen Bewegungsebene vorstehender Verbindungszapfen 15 mit gemäß Figur 3 unrundem Querschnitt in einem seitlich randoffenen Einführungsschlitz 16 der Lasche 8 befindet.

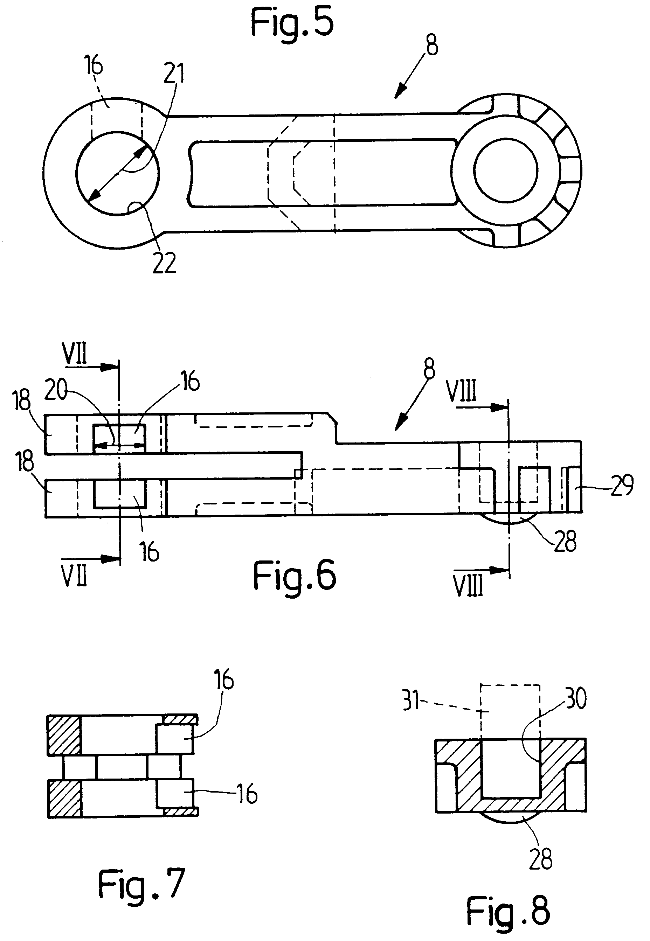

Beim Ausführungsbeispiel steht der Verbindungszapfen 15 beidseitig über den Grundkörpber 17 des Hebels 10 vor. Infolgedessen besteht auch der Einführungsschlitz 16 aus zwei einander gegenüberliegenden Schlitzteilen (Figur 6). Diese befinden sich ihrerseits an einem gegabelten Ende 18 der Lasche 5, welche selbstverständlich Bestandteil des Drehgelenks 11 ist. Gemäß Figur 3 besitzt der Verbindungszapfen 15 zwei parallele Abflachungen, deren Abstand die Breite 19 des Zapfens bestimmt, und welche der Schlitzbreite 20 (Figur 6) entspricht. Somit kann der Verbindungszapfen 15 senkrecht zur Bildebene der Figur 6 in den Schlitz 16 eingeschoben werden, wobei die Lasche 8 und der Hebel 10 dabei eine gestreckte Lage einnehmen. Sobald diese durch Drehen wenigstens eines der beiden Teile verändert wird, kann der Verbindungszapfen 15 den Einführungsschlitz 16 nicht mehr verlassen, und dies ist in sämtlichen Betriebsstellungen des Kurbeltriebs 9 der Fall. Damit aber der Verbindungszapfen 15 relativ zur Lasche 8 gedreht werden kann, entspricht der Durchmesser 21 der Bohrung 22 am inneren Ende des Einführungsschlitzes 16 dem Durchmesser 23 des Verbindungszapfens 15.

An seinem anderen Ende 24 trägt der Hebel 10 eine Hülse 25. Beim Ausführungsbeispiel ist sie mit einer Sackbohrung 26 ausgestattet, welche das innere Ende der erwähnten Wischerwelle drehfest aufnimmt. Gewissermaßen am Boden dieser Sackbohrung 16 befindet sich ein balliges Stützglied 27. Dieses liegt bei auf das Gehäuse 6 (Figur 1) aufgesetztem Deckel einer Innenfläche des letzteren an und bewirkt dadurch eine axiale Abstützung des Hebels 10.

Auch am wischwellenseitigen Ende der Lasche 8 ist ein balliges, innen am Deckel anliegendes bzw. anlegbares Stützglied angebracht, insbesondere angeformt, welches in den Figuren 6 und 8 mit 28 bezeichnet ist.

Am hebelfernen Ende 29 der Lasche 8 befindet sich beim Ausführungsbeispiel eine Bohrung 30 (Figur 8) zur Aufnahme des Lagerzapfens 7, welcher die Lasche 8 mit dem Getrieberad bzw. Schneckenrad 4 verbindet. Statt dessen kann man aber auch gemäß den gestrichelten Linien der Figur 8 einen Zapfen 31 an die Lasche 8 unmittelbar anformen, der dann den Lagerzapfen 7 der Figur 1 bildet. Dies wird insbesondere dann zweckmäßig sein, wenn man in bevorzugter Weise die Lasche 8 und den Hebel 10 aus Kunststoff herstellt, insbesondere als Spritzgußteil.

## Patentansprüche

1. Kurbeltrieb einer Antriebsvorrichtung eines Scheibenwischers für ein Kraftfahrzeug, bestehend aus einem Hebel (10) und einer über ein Drehgelenk (11) damit verbundenen Lasche (8), wobei die Lasche (8) exzentrisch (7) an einem angetriebenen Getrieberad, insbesondere Schneckenrad (4) des Getriebes, angelenkt ist und der Hebel (10) drehfest mit einer Wischerwelle (12) verbunden oder verbindbar ist, dadurch **gekennzeichnet**, daß die Lasche (8) und der Hebel (10) über eine bajonettartige Verbindung (13) gelenkig miteinander verbunden sind, wobei die Lasche (8) und der Hebel (10) in der Montagestellung eine gegenseitige Drehlage einnehmen, die von jeder Drehlage während des Betriebs verschieden ist.

2. Kurbeltrieb nach Anspruch 1, dadurch **gekennzeichnet**, daß die Lasche (8) und der Hebel (10) in der angekuppelten Montagestellung etwa in Verlängerung voneinander stehen.

3. Kurbeltrieb nach Anspruch 1 oder 2, dadurch **gekennzeichnet**, daß sich am einen Verbindungsende (14) des Hebels (10) ein quer zur Hebel-Bewegungsebene vorstehender Verbindungszapfen (15) mit unrundem Querschnitt befindet und die Lasche (8) am zugeordneten einen Verbindungsende (18) wenigstens einen seitlich randoffenen Einführungsschlitz (16) für den Verbindungszapfen (15) aufweist, der an seinem inneren Ende in eine durch einen Kreisbogen begrenzte, eine Lagerbohrung (21) bildende Schlitzerweiterung übergeht oder umgekehrt, und daß eine gedachte Umhüllende des Verbindungszapfens (15) einen Durchmesser (23) auf- weist, der demjenigen der Lagerbohrung (21) entspricht.

4. Kurbeltrieb nach Anspruch 3, dadurch **gekennzeichnet**, daß der Hebel (10) an seinem anderen Ende (24) eine insbesondere mit einer Sackbohrung (26) versehene Hülse (25) zur Aufnahme des inneren Endes der Wischerwelle aufweist.

5. Kurbeltrieb nach Anspruch 4, dadurch **gekennzeichnet**, daß sich am Hebel (10), der Hülse (25) etwa gegenüberliegend, ein balliges, innen an einem Gehäusedeckel der Antriebsvorrichtung abstützbares Abstützglied (27) befindet.

6. Kurbeltrieb nach wenigstens einem der Ansprüche 3 bis 5, dadurch **gekennzeichnet**, daß die Lasche (8) an ihrem Verbindungsende (18) mit dem Hebel (10) gabelartig gestaltet ist, wobei das zugeordnete Hebelende (14) zwischen die Gabelzinken der Lasche (8) greift, und daß sich an jedem Gabelzinken ein randoffener Einführungsschlitz (16) befindet.

7. Kurbeltrieb nach wenigstens einem der vorhergehenden Ansprüche, dadurch **gekennzeichnet**, daß sich am wischwellenseitigen Ende der Lasche (8) ein balliges, innen an einem Gehäusedeckel der Antriebsvorrichtung abstützbares Stützglied (28) befindet.

8. Kurbeltrieb nach wenigstens einem der vorhergehenden Ansprüche, dadurch **gekennzeichnet**, daß sich am hebelfernen Ende (29) der Lasche (8) ein quer zu ihrer Bewegungsebene vorstehender Lagerzapfen (31) zum Eingriff in eine Lagerbohrung am Zahnrad, insbesondere Schneckenrad (4), befindet.

9. Kurbeltrieb nach wenigstens einem der vorhergehenden Ansprüche, dadurch **gekennzeichnet**, daß der Hebel (10) und die Lasche (8) aus Kunststoff gefertigt, insbesondere gespritzt sind.

## Claims

1. A crank mechanism of a driving assembly of a screen wiper for an automotive vehicle, comprising a lever (10) and a tongue (8) connected to the lever by way of a swivel joint (11), with the tongue (8) being eccentrically (7) hinged to an actuated gear wheel, in particular, a worm gear wheel (4), and the lever (10) being connected or connectible in a non-rotating way to a wiper shaft (12),
characterized in that the tongue (8) and the lever (10) are pivotally interconnected by a bayonet-type connection (13), with the tongue (8) and the lever (10), in the assembled position, taking a mutual rotary position which is different from each rotary position during operation.

2. A crank mechanism as claimed in claim 1,
characterized in that the tongue (8) and the lever (10), in the coupled mounting condition, are approximately in extended relationship with respect to one another.

3. A crank mechanism as claimed in claim 1 or claim 2,
characterized in that provided on one of the connecting ends (14) of the lever (10) is a connecting pin (15) of an out-of-round cross-section projecting in a direction transverse to the plane of movement of the lever, and in that the tongue (8) on the associated connecting end (18) comprises at least one introduction slot (16) for the connecting pin (15), which is laterally open at the edge, with the connecting pin on the inner end thereof passing to a slot extension limited by a circular arc and forming a bearing bore (21), or vice versa, and in that an imaginary envelope of the connecting pin (15) has a diameter (23) corresponding to the diameter of the bearing bore (21).

4. A crank mechanism as claimed in claim 3,
characterized in that the lever (10) on the other end (24) thereof comprises a sleeve (25), in particular, provided with a blind bore (26) for accommodating the inner end of the wiper shaft.

5. A crank mechanism as claimed in claim 4,
characterized in that a cambered support member (27) supportable inwardly on a housing lid of the driving assembly is provided on lever (10) approximately opposite the sleeve (25).

6. A crank mechanism as claimed in at least one of claims 3 to 5,
characterized in that the tongue (8) at its end (18) connected to the lever (10) is of a bifurcated configuration, with the associated lever end (14) gripping between the fork spikes of the tongue (8), and in that provided on each fork spike is an introduction slot (16) open at the edge.

7. A crank mechanism as claimed in at least one of the preceding claims,
characterized in that provided on the end of the tongue (8) facing the wiper shaft is a cambered supporting member (28) inwardly supportable on a housing lid of the driving assembly.

8. A crank mechanism as claimed in at least one of the preceding claims,
characterized in that provided on the end (29) of the tongue (8) facing away from the lever is a bearing pin (31) protruding in a direction transverse to the plane of movement of the tongue for engagement with a bearing bore on the toothed gear wheel, preferably a worm gear wheel (4).

9. A crank mechanism as claimed in at least one of the preceding claims,
characterized in that the lever (10) and the tongue (8) are made of plastic material, in particular, by injection moulding.

## Revendications

1. Mécanisme à bielle et manivelle d'un dispositif d'entraînement d'essuie-glace pour véhicule automobile, constitué d'un levier (10) et d'une barre (8) reliée à ce levier par une articulation en rotation (11), la barre (8) étant articulée d'une manière excentrée (7) sur une roue d'engrenage entraînée, notamment une roue tangente (4), de l'engrenage et le levier (10) étant calé ou agencé de façon à pouvoir être calé sur un arbre d'essuie-glace (12), caractérisé en ce que la barre (8) et le levier (10) sont reliés l'un à l'autre d'une manière articulée au moyen d'une liaison (13) du type à baïonnette, la barre (8) et le levier (10) occupant dans la position de montage une position angulaire relative qui est différente de toute position angulaire occupée pendant le fonctionnement.

2. Mécanisme à bielle et manivelle selon la revendication 1, caractérisé en ce que la barre (8) et le levier (10) sont situés pratiquement dans le prolongement l'un de l'autre dans la position de montage accouplée.

3. Mécanisme à bielle et manivelle selon la revendication 1 ou 2, caractérisé en ce qu'il est prévu, situé à l'une (14) des extrémités de liaison du levier (10), un tourillon de liaison (15) à section transversale non circulaire qui se dresse transversalement au plan du mouvement du levier et la barre (8) comporte, à l'une (18), associée, de ses extrémités de liaison, au moins une fente (16) d'introduction du tourillon de liaison (15) qui est latéralement ouverte sur le bord et qui, à son extrémité intérieure, se raccorde à un élargissement de fente délimité par un arc de cercle et formant un alésage de palier (21), ou vice versa, et en ce qu'une surface-enveloppe imaginaire du tourillon de liaison (15) a un diamètre (23) qui est égal à celui de l'alésage de palier (21).

4. Mécanisme à bielle et manivelle selon la revendication 3, caractérisé en ce qu'à son autre extrémité (24), le levier (10) comporte une douille (25), notamment pourvue d'un alésage borgne (26), qui sert à recevoir l'extrémité intérieure de l'arbre d'essuie-glace.

5. Mécanisme à bielle et manivelle selon la revendication 4, caractérisé en ce qu'il est prévu, réalisé sur le levier (10) et d'une manière sensiblement opposée à la douille (25), un organe d'appui (27) de forme bombée qui est agencé de façon à pouvoir prendre appui intérieurement sur un couvercle de boîtier du dispositif d'entraînement.

6. Mécanisme à bielle et manivelle selon au moins l'une des revendications 3 à 5, caractérisé en ce que la barre (8) est réalisée en forme de fourche à son extrémité (18) de liaison avec le levier (10), l'extrémité associée (14) de ce levier s'engageant entre les branches de fourche de la barre (8), et en ce qu'une fente d'introduction (16) ouverte sur le bord est ménagée sur chaque branche de la fourche.

7. Mécanisme à bielle et manivelle selon au moins l'une des revendications précédentes, caractérisé en ce qu'il est prévu, disposé à l'extrémité de la barre (8) qui est située du côté de l'arbre d'essuie-glace, un organe d'appui (28) de forme bombée qui est agencé de façon à pouvoir prendre appui intérieurement sur un couvercle de boîtier du dispositif d'entraînement.

8. Mécanisme à bielle et manivelle selon au moins l'une des revendications précédentes, caractérisé en ce qu'il est prévu, disposé à l'extrémité (29) de la barre (8) qui est éloignée du levier, un tourillon de palier (31) qui se dresse transversalement à son plan de mouvement et est destiné à s'engager dans un alésage de palier pratiqué dans la roue dentée, notamment la roue tangente (4).

9. Mécanisme à bielle et manivelle selon au moins l'une des revendications précédentes, caractérisé en ce que le levier (10) et la barre (8) sont réalisés en matière plastique, notamment par injection.
